# EUROPEAN PATENT APPLICATION

(11) **EP 2 039 383 A1**
(43) Date of publication of application: **25.03.2009**
(21) Application number: 07253701.2
(22) Date of filing: 18.09.2007
(51) Int. Cl.: A61M 5/32, A61M 5/50

(54) **Disposable syringe guarded in a preuse position**

(71) Applicant: Shue, Ming-Jeng, Hsi Dist, Taichung City (TW); Shue, Phillip, Hsi District, Taichung City (TW); Huang, Deborah, Chung Dist Taichung City (TW)
(72) Inventor: Shue, Ming-Jeng, Hsi Dist, Taichung City (TW); Shue, Phillip, Hsi District, Taichung City (TW); Huang, Deborah, Chung Dist Taichung City (TW)
(74) Representative: Mackenzie, Andrew Bryan

(57) **Abstract**

A disposable syringe includes a barrel (1), a needle assembly (2), a plunger (3), a press-fitting unit (51,52), and a spacer (4). The plunger (3) is movable in the barrel (1) to a retraction initiating position, where a front open plunger end (37) is coupled with the needle assembly (2) for withdrawal of the needle assembly (2) into the plunger (3). An endcap (33) is disposed to cover a rear open plunger end (38) of the plunger (3). The press-fitting unit (51,52) is disposed between the plunger (3) and an inner barrel surface (130) of the barrel (1) to permit press-fit engagement between the plunger (3) and the barrel (1) immediately after the plunger (3) is moved to the retraction initiating position. The spacer (4) includes front and rear abutment surfaces (411,412) detachably engaged with the barrel (1) and the endcap (33) in a preuse position.

## Description

This invention relates to a disposable syringe, more particularly to a disposable syringe which has a spacer to guard a plunger against undesired movement to a needle retraction initiating position so as to guard the syringe in a preuse position.

Conventional medical devices or syringes for injection medicine, drawing blood samples, etc., have to be disposed of safely after injection in order to avoid accidental needle pricks or undesirable contamination. Although a tip protector is provided to be sleeved on the syringe after use to ensure that the needle is covered, the user is exposed to the risk of being pricked by the needle when sleeving the tip protector on the syringe. Therefore, there are available medical devices or syringes with a retractable needle that is retractable into a barrel or a plunger after the injection operation is completed, such as those disclosed in U.S. Patent Nos. 6,743,199 and 6,921,386 issued to the applicants, and U.S. Patent Application Nos. 10/918,020and 11/320,927 filed by the applicants. However, further improvements are desirable to prevent undesired withdrawal of the needle caused by inadvertent pressing of the plunger in a preuse position, to ensure successful withdrawal of the needle, and to have a slenderized construction for small volume injection.

One object of the present invention is to provide a disposable syringe which can be guarded in a preuse position to prevent undesired withdrawal of a needle caused by inadvertent pressing of the plunger in the preuse position.

Another object of the present invention is to provide a disposable syringe which can ensure successful withdrawal of a needle.

Still another object of the present invention is to provide a disposable syringe which has a slenderized construction for small volume injection.

According to this invention, the disposable syringe includes a barrel, a needle assembly, a plunger, a press-fitting unit, and a spacer. The barrel has front and rear open barrel ends opposite to each other along an axis, and a surrounding barrel wall which has an inner barrel surface that defines a passage. Apair of lugs extend radially from the surrounding barrel wall and adjacent to the rear open barrel end. The needle assembly includes a needle cannula and a needle seat to secure the needle cannula. The needle seat is movable in the passage between a position of use, where a tip end of the needle cannula extends outwardly of the front open barrel end, and a disposal position, where the tip end of the needle cannula retreats in the passage. The plunger has a front open plunger end, a rear open plunger end which extends outwardly of the rear open barrel end, and an intermediate surrounding wall which defines an accommodation chamber, and which has first and second rear segments. The plunger is movable in the passage between the position of use, and a retraction initiating position, where the front open plunger end is coupled with the needle seat for withdrawal of the needle seat together with the needle cannula into the accommodation chamber. An endcap has an endwall extending radially to cover the rear open plunger end, and a ring wall extending from a periphery of the endwall to surround the first rear segment and terminate at a rim that confronts the lugs. A press-fitting unit is disposed between the second rear segment and the inner barrel surface such that immediately after the plunger is moved to the retraction initiating position, the intermediate surrounding wall is brought into press-fit engagement with the inner barrel surface. The spacer includes front and rear abutment surfaces which are detachably engaged with the lugs and the rim, respectively, so as to guard the plunger against moving towards the retraction initiating position.

Other features and advantages of the present invention will become apparent in the following detailed description of the preferred embodiments of the invention, with reference to the accompanying drawings, in which:
Fig. 1 is a sectional view of the first preferred embodiment of a disposable syringe according to this invention;
Fig. 2 is a fragmentary sectional view of a front part of the first preferred embodiment;
Fig. 3 is a fragmentary sectional view of a rear part of the first preferred embodiment;
Fig. 4 is a fragmentary perspective view of a spacer and a pair of lugs of the first preferred embodiment;
Fig. 5 is a sectional view of the first preferred embodiment, showing how the spacer guards against movement of a plunger;
Fig. 6 is a sectional view of the first preferred embodiment in a retracted state;
Fig. 7 is a sectional view of the first preferred embodiment in a modified form;
Fig. 8 is a fragmentary sectional view of a rear part of the second preferred embodiment of a disposable syringe according to this invention;
Fig. 9 is a fragmentary sectional view of a rear part of the second preferred embodiment, showing how a spacer guards against movement of a plunger;
Fig. 10 is a fragmentary sectional view of a rear part of the third preferred embodiment of a disposable syringe according to this invention, showing how a spacer guards against movement of a plunger;
Fig. 11 is a fragmentary sectional view of the rear part of the third preferred embodiment, showing that the spacer is removed; and
Fig. 12 is a sectional view of the fourth preferred embodiment of a disposable syringe according to this invention in a state of use.

Before the present invention is described in greater detail, it should be noted that same reference numerals have been used to denote like elements throughout the specification.

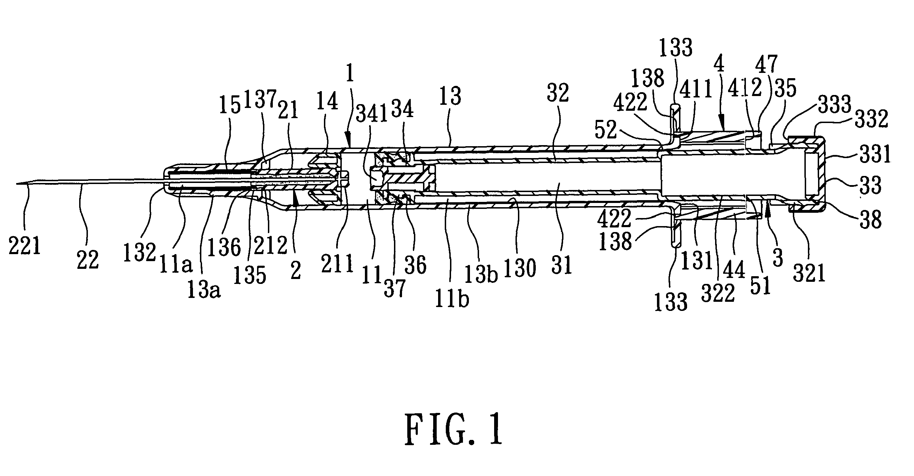

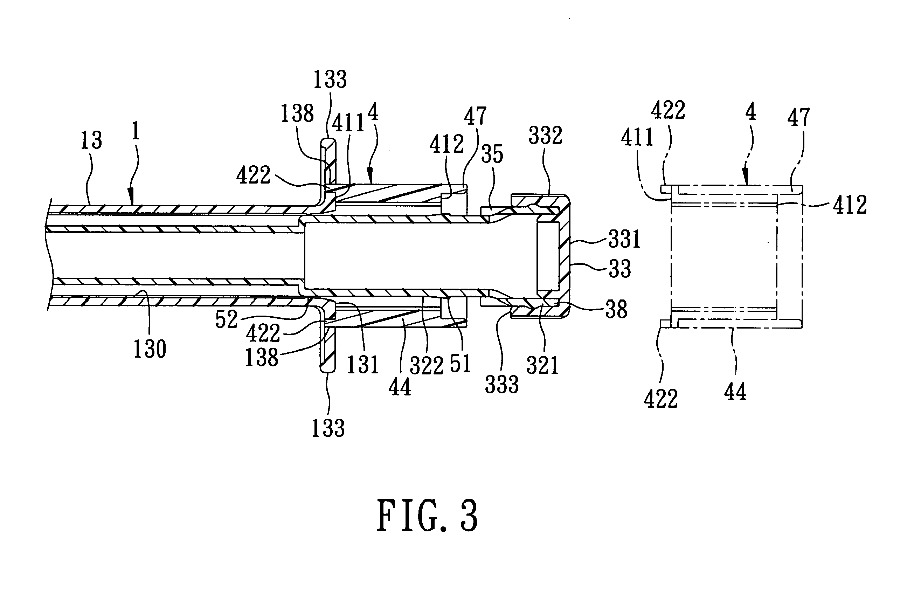

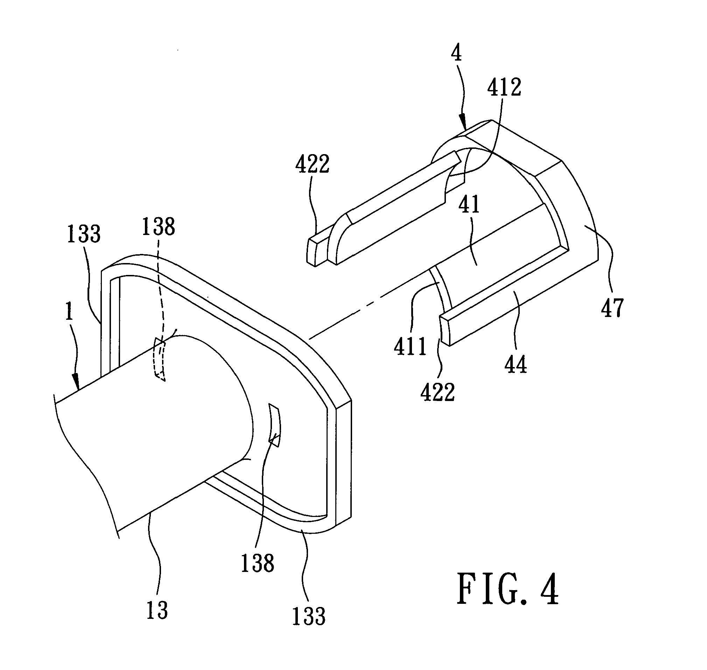

Referring to Figs. 1 to 4, the first preferred embodiment of a disposable syringe according to the present invention is shown to comprise a barrel 1, a needle assembly 2, a plunger 3, a press-fitting unit, and a spacer 4.

The barrel 1 has front and rear open barrel ends 132, 131 opposite to each other along an axis, and a surrounding barrel wall 13 which interconnects the front and rear open barrel ends 132, 131, and which has an inner barrel surface 130 that defines a passage 11. The surrounding barrel wall 13 has small-diameter and large-diameter wall portions (13a,13b) which are disposed proximate to the front and rear open barrel ends 132,131, respectively, to divide the passage 11 into small and large passage segments (11a,11b). The small-diameter wall portion (13a) has a flange 136 confronting rearwardly, and a plurality of retaining ribs 137 disposed adjacent to the large-diameter wall portion (13b). A pair of lugs 133 extend radially from the large-diameter wall portion (13b) and adjacent to the rear open barrel end 131, and have insertion slots 138.

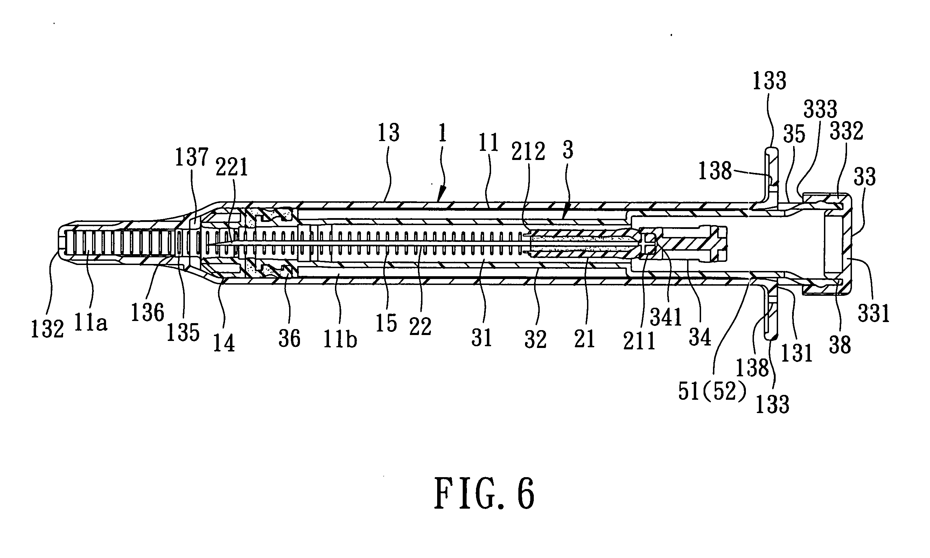

The needle assembly 2 includes a needle cannula 22 and a needle seat 21. The needle seat 21 is configured to secure the needle cannula 22, and is disposed to be movable in the passage 11 between a position of use, as shown in Fig. 1, where a tip end 221 of the needle cannula 22 extends outwardly of the front open barrel end 132, and a disposal position, as shown in Fig. 6, where the tip end 221 of the needle cannula 22 is retracted into the passage 11. The needle seat 21 has a rear seat portion 213 which is retained in the large passage segment (11b) by means of a grip member 14 so as to be placed in the position of use, and a front seat portion 214 which extends in the small passage segment (11a) and which is in frictional engagement with the small-diameter wall portion (13a) and the retaining ribs 137. The grip member 14 is in slidable air-tight engagement with the large-diameter wall portion (13b) and the rear seat portion 213, and is spaced apart from the small passage segment (11a) so as to be movable forwardly in the large passage segment (11b). The front seat portion 214 has a front abutment 212 which is spaced apart from the flange 136 along the axis in the position of use so as to define a triggering space 135 therebetween.

A coil spring 15 is disposed in the small passage segment (11a) to surround the needle cannula 22, and has front and rear biasing ends that abut against the front open barrel end 132 and the front seat portion 214, respectively, so as to be placed in a compressed state when the needle seat 21 is in the position of use.

The needle seat 21 further has an anchored portion 211 which extends rearwardly from the rear seat portion 213 along the axis. In this embodiment, the anchored portion 211 is in the form of a plug. The plug has an axial passageway 2111 and two transverse passageways 2112, which are fluidly communicated with the passage 11 and the needle cannula 22, and which respectively extend along the axis and transverse to the axis.

The plunger 3 has a front open plunger end 37 which is spaced apart from the needle seat 21, and a rear open plunger end 38 which is opposite to the front open plunger end 37 along the axis, and which extends outwardly of the rear open barrel end 131 of the barrel 1. The plunger 3 further has an intermediate surrounding wall 32 which is interposed between the front and rear open plunger ends 37, 38 to define an accommodation chamber 31 therebetween, and which has first and second rear segments 321,322 respectively proximate to and distal from the rear open plunger end 38, and a protrusion 35 between the first and second rear segments 321,322. A seal member 36 is retainingly sleeved on the front open plunger end 37, and is air-tightly and slidably engaged with the inner barrel surface 130 of the large-diameter wall portion (13b). A coupling rod 34 is detachably inserted into the front open plunger end 37 to close the accommodation chamber 31. The coupling rod 34 has an anchoring area 341 in the form of a socket that is configured to engage and mate with the plug, i.e. the anchored portion 211.

An endcap 33 has an endwall 331 which extends in a transverse direction relative to the axis to cover the rear open plunger end 38, and a ring wall 332 which extends from a periphery of the endwall 331 along the axis to surround the rear segment 321 and to terminate at a rim 333 that confronts the lugs 133.

The press-fitting unit includes protrusions 51, 52 which are disposed on the second rear segment 322 of the plunger 3 and the inner barrel surface 130 of the large-diameter wall portion (13b).

The spacer 4 includes a spacer body 41 which has front and rear abutment surfaces 411,412 opposite to each other along the axis, two pins 422 which are disposed forwardly of the front abutment surface 411, and an abutment segment 47 which is disposed rearwardly of the rear abutment surface 412, which is outboard to the ring wall 332, and which is aligned with the pins 422. The abutment segment 47 is configured to extend angularly about the axis. The spacer 4 further includes a ridge 44 which is disposed between the abutment segment 47 and the pins 422 and which extends along the axis to the spacer body 41 for easy manipulation.

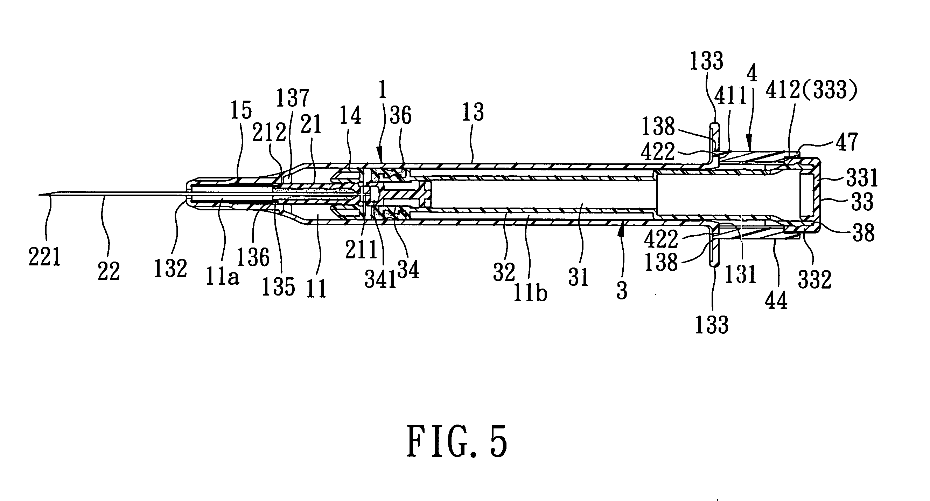

Referring to Fig. 5, the front and rear abutment surfaces 411,412 of the spacer 4 are engaged with the lugs 133 and the rim 333 of the endcap 33, respectively, and the pins 422 are inserted in the insertion slots 138, respectively, in a preuse position so as to guard the plunger 3 against forward movement towards a retraction initiating position, where the anchoring area 341 is engaged with the anchored portion 211. By insertion of the pins 422 into the insertion slots 138, slipping of the front abutment surface 411 out of engagement with the lugs 133 can be prevented. In addition, the rear abutment surface 412 is prevented by the abutment segment 47 from slipping from engagement with the rim 333.

Referring to Fig. 5, before an injection operation, the user can press the plunger 3 forwards to substantially eliminate adhesion between the plunger 3 and the barrel 1 or to draw medicine liquid. By providing the spacer 4, the front open plunger end 37 can be kept away from the retraction initiating position so as to prevent undesired withdrawal of the needle cannula 22 caused by inadvertent pressing of the plunger 3, thereby ensuring the effectiveness of the disposable syringe.

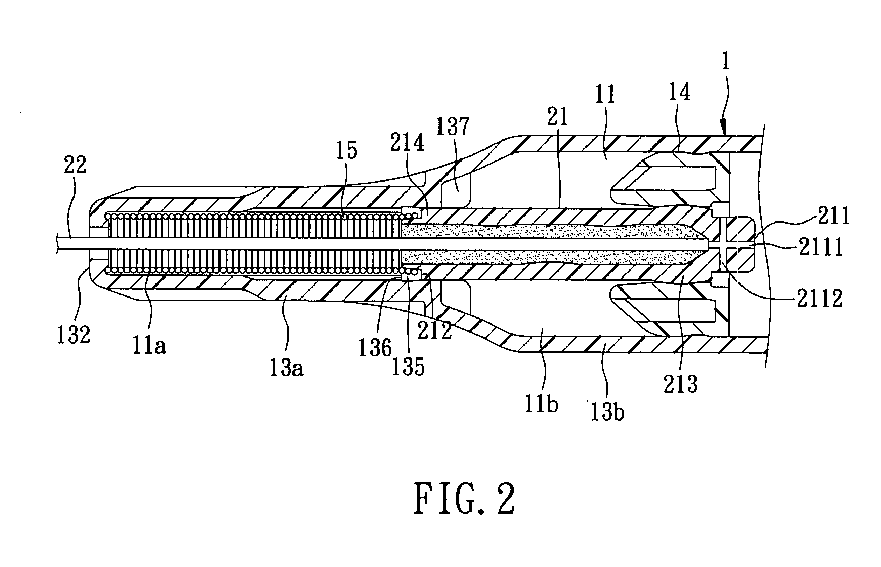

Referring to Figs. 2, 5 and 6, during an injection operation, the user first removes the spacer 4 by pressing the ridge 44 with his/her finger. The plunger 3 is then pressed forwards until the seal member 36 abuts against the grip member 14, thereby completing the injection and placing the plunger 3 in the retraction initiating position. At this time, the anchoring area 341 is matingly engaged with the anchored portion 211. Hence, liquid trapped in the anchoring area 341 and the large passage segment (11b) is permitted to enter the needle cannula 22 through the axial and transverse passageways 2111,2112. Moreover, the axial and transverse passageways 2111,2112 are closed by the anchoring area 341 when the anchored portion 211 is completely inserted into and is engaged with the anchoring area 341, thereby preventing splashing of the liquid from the needle cannula 22 during a subsequent disposal operation.

When the plunger 3 is further pressed forwards to move the grip member 14 and the needle seat 21 forwardly, the front abutment 212 of the needle seat 21 is moved into the triggering space 135 to abut against the flange 136. By means of the movement of the needle seat 21 and the grip member 14, the friction and adhesion of the needle seat 21 upon the small-diameter wall portion (13a) and the friction and sticking of the grip member 14 upon the large-diameter wall portion (13b) can be diminished to facilitate a subsequent withdrawal of the needle seat 21 together with the needle cannula 22 into the accommodation chamber 31. At the same time, the needle seat 21 is disengaged from the grip member 14, and the coupling rod 34 is forced to move rearwards to disengage from the front open plunger end 37 so as to allow the coil spring 15 to bias the needle seat 21, as well as the needle cannula 22, to the disposal position. Thus, successful and smooth withdrawal of the needle cannula 22 is ensured.

It is noted that, immediately after the plunger 3 is moved to the retraction initiating position, the intermediate surrounding wall 32 is brought into press-fit engagement with the inner barrel surface 130 by means of the protrusions 51,52 of the press-fitting unit so as to permit the plunger 3 to be retained in the large passage segment (11b), thereby preventing reuse of the syringe. In addition, once the protrusion 35 abuts against the rear open barrel end 131, the forward movement of the plunger 3 is checked so as to prevent application of an excess compression force to the grip member 14, which may result in deformation of the grip member 14 and may hence hinder retraction of the needle seat 21.

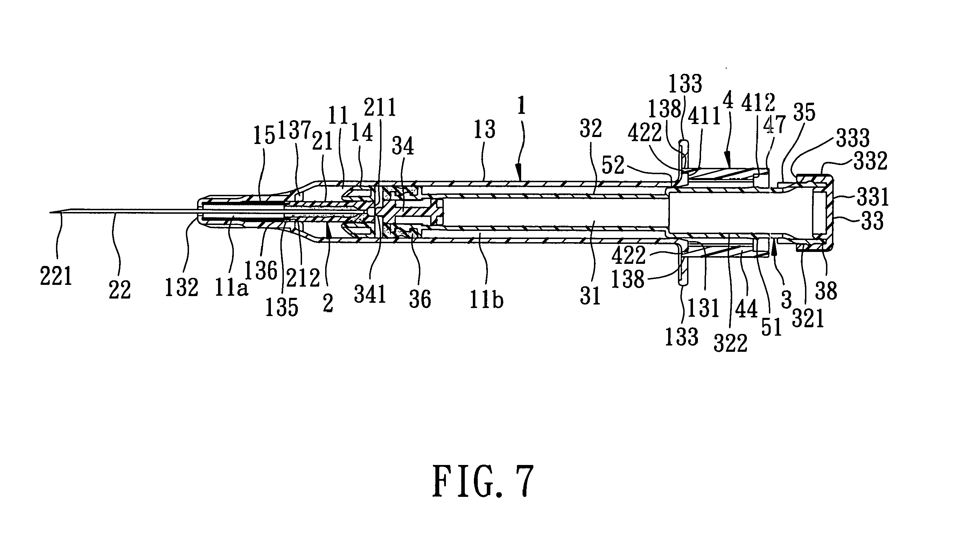

Referring to Fig. 7, alternatively, the anchored portion 211 and the anchoring area 341 are in the form of a socket and a plug, respectively, which can engage and mate with each other for withdrawal of the needle seat 21 together with the needle cannula 22 into the accommodation chamber 31.

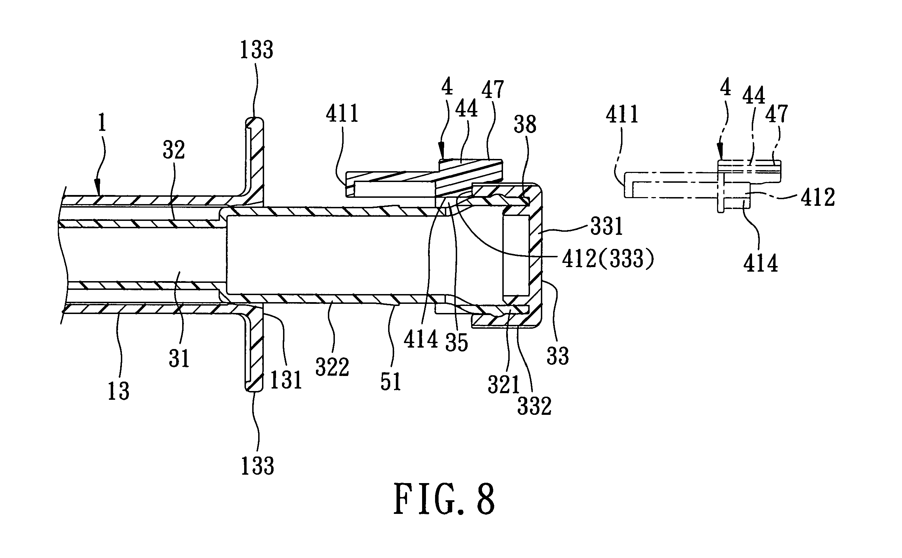

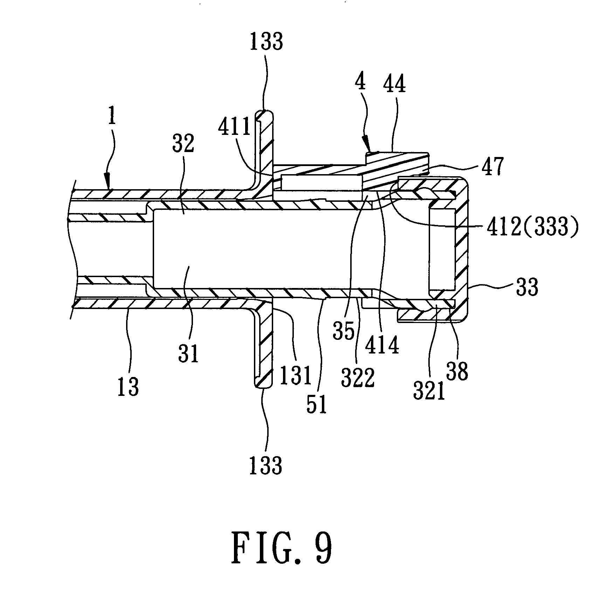

Referring to Figs. 8 and 9, the second preferred embodiment of a preuse-guarded disposable syringe according to this invention is similar to the first preferred embodiment in construction, except that there is provided a mortise joint, which has the protrusion 35 in the form of a tenon 35 disposed on the second rear segment 322 of the plunger 3, and a mortise 414 disposed in the spacer 4 for receiving the tenon 35 fittingly so as to prevent the spacer 4 to move angularly about the axis in the preuse position.

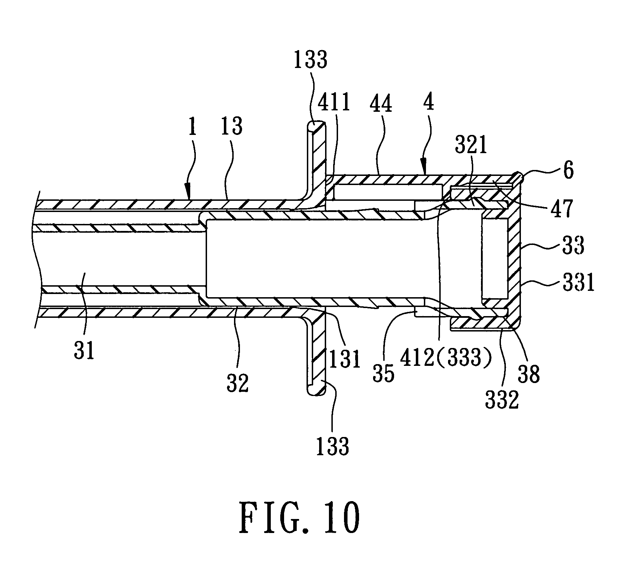

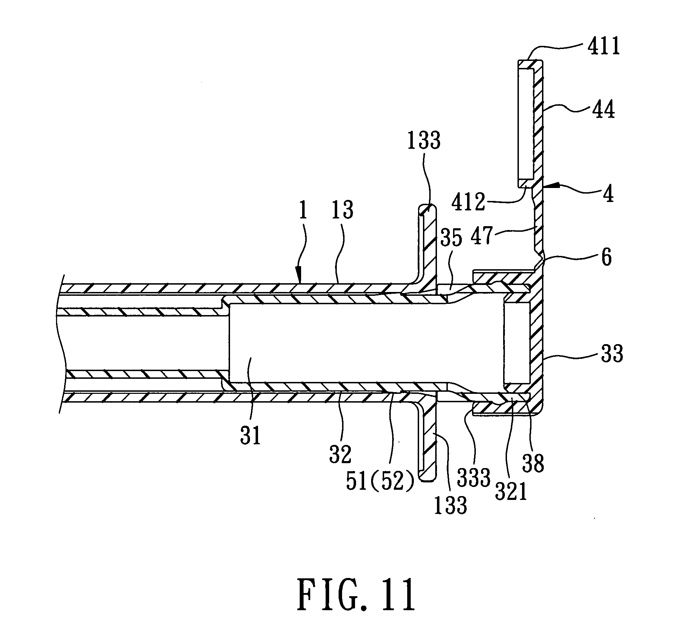

Referring to Figs. 10 and 11, the third preferred embodiment of a disposable syringe according to this invention is similar to the first preferred embodiment in construction, except that a flexible connecting strap 6 is further disposed to interconnect the endcap 33 and the abutment segment 47 of the spacer 4, and is configured such that the front and rear abutment surfaces 411,412 are permitted to be removed from the lugs 133 and the rim 333 of the endcap 33. In this embodiment, the flexible connecting strap 6 is integrally formed with the spacer 4 and the endcap 33.

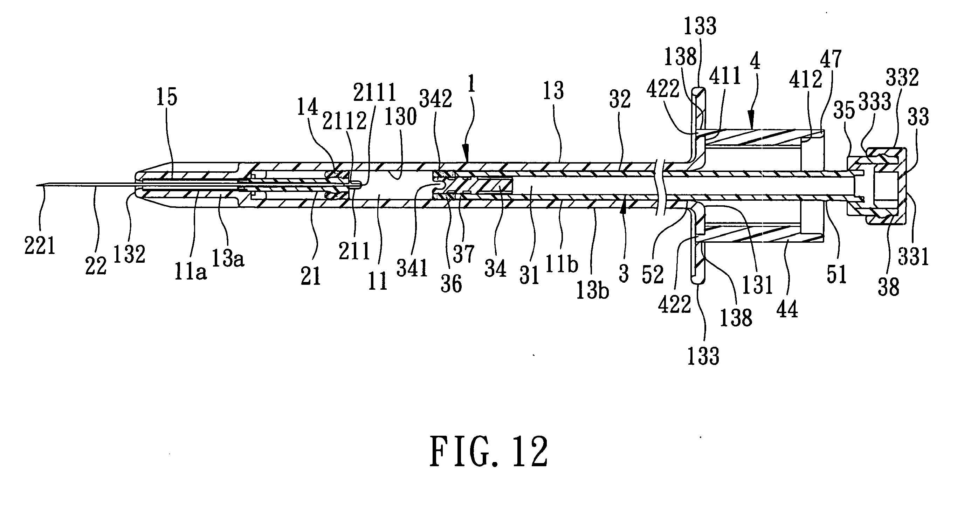

In the aforesaid embodiments, a disposable syringe is provided for injection of medication of a general volume, such as 3 ml, 5 ml or more. In the fourth preferred embodiment of this invention, as shown in Fig. 12, the disposable syringe is used for injection of medication of a very small volume, such as 1 ml or 0.5 ml. In other words, the barrel 1, the needle assembly 2, the plunger 3, and the spacer 4 have a relatively small structure.

In order to obtain a slenderized construction of a disposable syringe of the present invention, the coupling rod 34 has a front coupling portion 342 which extends forwardly beyond the front open plunger end 37 and which has an anchoring area 341 in the form of a socket. The seal member 36 is retainingly sleeved on the front coupling portion 342, and is air-tightly and slidably engaged with the inner barrel surface 130 of the large-diameter wall portion (13b).

It is noted that, since the needle seat 21 is movably engaged with the grip member 14 and is largely disposed in the large passage segment (11b) of the barrel 1, and since the coil spring 15 of this invention is disposed to surround the needle cannula 22 with the rear biasing end abutting against the front seat portion 214 of the needle seat 21, the diameter of the coil spring 15 and the diameter of the needle seat 21 can be relatively small. Thus, the inner diameter of the barrel 1 for accommodating the plunger 3, which in turn accommodates the retracted needle seat 21, can be made comparatively small so as to slenderize the whole construction of the disposable syringe for convenient detailed calibration of small volumes, such as 0.5 ml and 1 ml. In addition, compared with the syringes of the previous embodiments in which the seal member 36 is sleeved on the front open plunger end 37 of the plunger 3, the seal member 36 of this embodiment is sleeved on the front coupling portion 342 of the coupling rod 34, thereby maximizing the inner diameter of the front open plunger end 37 for facilitating entry of the retracted needle seat 21 into the accommodation chamber 31 of the comparatively slender plunger 3.

## Claims

1. A disposable syringe guarded in a preuse position, comprising:
a barrel (1) having front and rear open barrel ends (132,131) opposite to each other along an axis, and a surrounding barrel wall (13) which interconnects said front and rear open barrel ends (132,131), and which has an inner barrel surface (130) that defines a passage (11);
a pair of lugs (133) which extend radially from said surrounding barrel wall (13) and adjacent to said rear open barrel end (131);
a needle cannula (22);
a needle seat (21) which is configured to secure said needle cannula (22), and which is disposed to be movable in said passage (11) between a position of use, where a tip end (221) of said needle cannula (22) extends outwardly of said front open barrel end (132), and a disposal position, where said tip end (221) of said needle cannula (22) is retracted into said passage (11);
a plunger (3) having a front open plunger end (37) which is spaced apart from said needle seat (21), and a rear open plunger end (38) which is opposite to said front open plunger end (37) along the axis, and which extends outwardly of said rear open barrel end (131), said plunger (3) further having an intermediate surrounding wall (32) which is interposed between said front and rear open plunger ends (37,38) to define an accommodation chamber (31) therebetween, and which has first and second rear segments (321,322) respectively proximate to and distal from said rear open plunger end (38), said plunger (3) being movable in said passage (11) between the position of use, and a retraction initiating position, where said front open plunger end (37) is coupled with said needle seat (21) for withdrawal of said needle seat (21) together with said needle cannula (22) into said accommodation chamber (31) ; and
an endcap (33) which has an endwall (331) having a periphery and extending in a transverse direction relative to the axis to cover said rear open plunger end (38), and a ring wall (332) extending from said periphery of said endwall (331) along the axis to surround said first rear segment (321) and to terminate at a rim (333) that confronts said lugs (133), **characterized by:**
a press-fitting unit (51,52) disposed between said second rear segment (322) and said inner barrel surface (130) such that, immediately after said plunger (3) is moved to the retraction initiating position, said intermediate surrounding wall (32) is brought into press-fit engagement with said inner barrel surface (130) ; and
a spacer (4) including front and rear abutment surfaces (411, 412) which are opposite to each other along the axis, and which are detachably engaged with one of said lugs (133) and said rim (333), respectively, so as to prevent said plunger (3) from moving towards the retraction initiating position.

2. The disposable syringe according to Claim 1, **characterized in that** one of said lugs (133) has an insertion slot (138), said spacer (4) further including a pin (422) which is disposed forwardly of said front abutment surface (411) to be inserted into said insertion slot (138) so as to prevent said front abutment surface (411) from slipping out of engagement with a corresponding one of said lugs (133).

3. The disposable syringe according to Claim 2, **characterized in that** said spacer (4) includes an abutment segment (47) which is disposed rearwardly of said rear abutment surface (412) and which is outboard to said ring wall (332), said abutment segment (47) being aligned with said pin (422) such that, in the preuse position, said rear abutment surface (412) is prevented by said abutment segment (47) from slipping from engagement with said rim (333).

4. The disposable syringe according to Claim 1, **characterized in that** said spacer (4) includes an abutment segment (47) which is disposed rearwardly of said rear abutment surface (412) and which is outboard to said ring wall (332).

5. The disposable syringe according to Claim 4, further **characterized by** a mortise joint which has a tenon (35) disposed on said second rear segment (322) of said plunger (3), and a mortise (414) disposed in said spacer (4) for receiving said tenon (35) fittingly so as to prevent said spacer (4) from moving angularly about the axis in the preuse position.

6. The disposable syringe according to Claim 4, **further characterized by** a flexible connecting strap (6) which interconnects said endcap (33) and said abutment segment (47) and which is configured such that said front and rear abutment surfaces (411,412) are permitted to be removed from one of said lugs (133) and said rim (333) .

7. The disposable syringe according to Claim 1, **further characterized by** a coil spring (15) which is disposed between said needle seat (21) and said front open barrel end (132) and which surrounds said needle cannula (22) so as to be placed in a compressed state when said needle seat (21) is in the position of use.

8. The disposable syringe according to Claim 1, **characterized in that** said surrounding barrel wall (13) has small-diameter and large-diameter wall portions (13a,13b) which are disposed proximate to said front and rear open barrel ends (132,131), respectively, to divide said passage (11) into small and large passage segments (11a,11b), said needle seat (21) having a rear seat portion (213) which is retained in said large passage segment (11b) so as to be placed in the position of use, and a front seat portion (214) which extends in said small passage segment (11a) and which is in frictional engagement with said small-diameter wall portion (13a), said disposable syringe further comprising a coil spring (15) which has front and rear biasing ends that abut against said front open barrel end (132) and said front seat portion (214), respectively, so as to be placed in a compressed state when said needle seat (21) is in the position of use.

9. The disposable syringe according to Claim 8, **characterized in that** said small-diameter wall portion (13a) has a flange (136) which confronts rearwardly, said front seat portion (214) having a front abutment (212) which is spaced apart from said flange (136) along the axis in the position of use so as to define a triggering space (135) therebetween, such that, by virtue of forward movement of said plunger (3) to couple said front open plunger end (37) with said needle seat (21), said front abutment (212) is moved into said triggering space (135) to abut against said flange (136).

10. The disposable syringe according to Claim 8, **further**
**characterized by** a grip member (14) which is in slidable air-tight engagement with said large-diameter wall portion (13b) and said rear seat portion (213) and which is spaced apart from said small passage segment (11a) so as to be movable forwardly in said large passage segment (11b) .

11. The disposable syringe according to Claim 8,
**characterized in that** said needle seat (21) has an anchored portion (211) which extends rearwardly from said rear seat portion (213) along the axis, said disposable syringe further comprising a coupling rod (34) which is detachably inserted into said front open plunger end (37) to close said accommodation chamber (31), and which has an anchoring area (341) that is engageable with said anchored portion (211) so as to couple said plunger (3) with said needle seat (21) for withdrawal of said needle seat (21).

12. The disposable syringe according to Claim 11,
**characterized in that** said anchored portion (211) and said anchoring area (341) are, respectively, a plug and a socket which engage and mate with each other, said plug having axial and transverse passageways (2111,2112) which are fluidly communicated with said passage (11) and said needle cannula (22), and which respectively extend along the axis and transverse to the axis, such that liquid trapped in said socket and said passage (11) is permitted to enter said needle cannula (22) through said axial and transverse passageways (2111,2112), respectively, when said plug is inserted into said socket, and such that said axial and transverse passageways (2111,2112) are closed by said socket when said plug is completely inserted into and is engaged with said socket so as to prevent splashing of the liquid from said needle cannula (22).

13. The disposable syringe according to Claim 12,
**characterized in that** said coupling rod (34) has a front coupling portion (342) which extends forwardly beyond said front open plunger end (37) and which defines said socket therein, said disposable syringe further comprising a seal member (36) which is retainingly sleeved on said front coupling portion (342) and which is air-tightly and slidably engaged with said inner barrel surface (130) of said large-diameter wall portion (13b) .

14. The disposable syringe according to Claim 11,
**characterized in that** said anchored portion (211) and said anchoring area (341) are, respectively, a socket and a plug which engage and mate with each other.

15. The disposable syringe according to Claim 11, **further characterized by** a seal member (36) which is retainingly sleeved on said front open plunger end (37) and which is air-tightly and slidably engaged with said inner barrel surface (130) of said large-diameter wall portion (13b).

16. The disposable syringe according to Claim 1,
**characterized in that** said intermediate surrounding wall (32) has a protrusion (35) which is disposed to abut against said rear open barrel end (131) so as to prevent excess forward movement of said plunger (3).
